# EUROPEAN PATENT SPECIFICATION

(11) **EP 3 003 047 B1**
(45) Date of publication and mention of the grant of the patent: **15.08.2018**
(21) Application number: 14726619.1
(22) Date of filing: 28.05.2014
(51) Int. Cl.: A01N 63/00, A01P 21/00

(54) **MICROBIAL AGRICULTURE**
MIKROBIELLE LANDWIRTSCHAFT
AGRICULTURE MICROBIENNE

(30) Priority: 31.05.2013 US 201361830025 P; 27.06.2013 US 201361840118 P
(43) Date of publication of application: 13.04.2016
(73) Proprietor: DSM IP Assets B.V., 6411 TE Heerlen (NL)
(72) Inventor: DONNERS, Ruth Emelia Wilhelmina, NL-6100 AA Echt (NL); KUMAR, Manoj, NL-6100 AA Echt (NL)
(74) Representative: DSM Intellectual Property
(86) International application number: PCT/EP2014/061030
(87) International publication number: WO 2014/191450

(56) References cited:
- WO-A1-93/01720
- WO-A1-2004/087934
- US-A- 5 496 547

## Description

### Field of the invention

The present invention discloses new antimicrobial compositions to control plant diseases and to prevent microbial spoilage of crops.

### Background of the invention

Pathogenic microorganisms affecting plant health are a major and chronic threat to food production and ecosystem stability worldwide. Not only from an economical point of view, but also from a humane point of view it is of great importance to prevent spoilage of food products. After all, in many parts of the world people suffer from hunger.

As agricultural production intensified over the past few decades, producers became more and more dependent on agrochemicals such as fungicides as a relatively reliable method of crop protection helping with economic stability of their operations. However, increasing use of fungicides causes several negative effects, i.e., development of pathogen resistance to the applied fungicide and their non-target environmental impacts. Furthermore, the growing cost of fungicides, particularly in less-affluent regions of the world, and consumer demand for pesticide-free food has led to a search for substitutes for these products. There are also a number of fastidious diseases for which chemical solutions are few, ineffective, or non-existent. Biological control is thus being considered as an alternative or a supplemental way of reducing the use of chemicals in agriculture.

Over the past forty years many new fungicides have been developed and marketed. However, these fungicides have not been immune to challenges in their development and maintenance. A large concern has been resistance development. Resistance to fungicides has been observed on several diseases now.

Consequently, it can be concluded that there is an urgent need to develop alternatives to fungicides for the control of plant diseases. These alternatives should be developed with sustainable agriculture in mind, replacing conventional fungicides and helping to fight the growing problem of resistance.

### Description of the figures

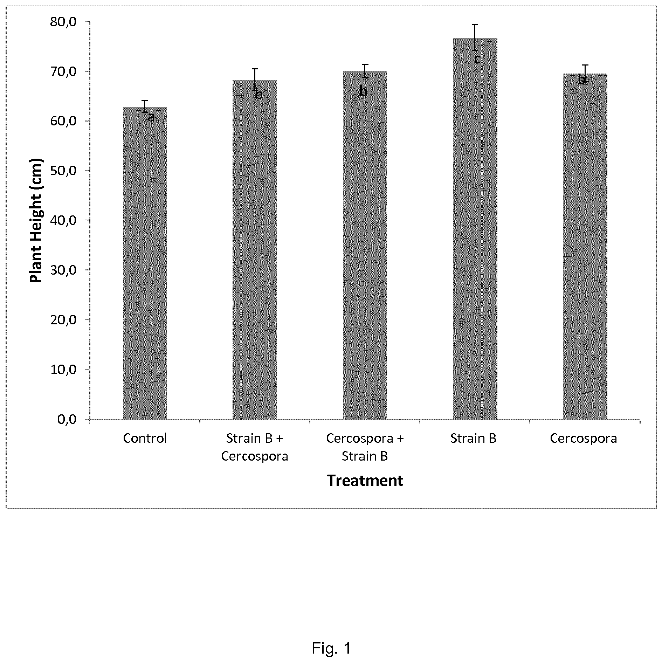

Figure 1: Plant height of corn at 84 days after planting/42 days after inoculation with natamycin producing strain ATCC27448 (called Strain B in Figure 1) and/or *Cercospora zeae-maydis.* Different letters (a,b,c) mean significantly different results at α=0.05).

### Description of the invention

The present invention solves the problem by providing natamycin producing bacterial strains.

In an embodiment the present invention relates to a method for enhancing plant growth, crop yield or both, the method comprising the step of applying at least one natamycin producing bacterial strain to a plant.

By applying the natamycin producing bacterial strain to a plant, a positive yield response in the plant may be obtained. Moreover, the quality of harvested plant materials may be improved by application of the natamycin producing bacterial strain to the plant, as the contents of mycotoxins in plants and/or harvested plant materials may be reduced.

"Plant" as used herein means that the natamycin producing bacterial strain can be applied to a whole plant or part thereof. So, in an embodiment of the present invention the natamycin producing bacterial strain can be applied to a whole plant or part thereof. Plants that can be treated vary from wild plants (wanted or unwanted) to crop plants (e.g. crop plants obtained by conventional breeding and optimisation techniques (e.g. crossing or protoplast fusion), by biotechnological and genetic engineering techniques (e.g. mutagenesis or recombinant DNA techniques) or by a combination thereof). Part of plants includes all aboveground and below-ground parts and organs of plants. For instance, the natamycin producing bacterial strain can be applied to roots, shoots, tubers, rhizomes, seedlings, stems, foliage, cuttings, needles, stalk, leaves, fruits, trunks flowers, to name just a few.

Preferably, the strain is living when applied. The strain may be applied in any physiological state such as active or dormant. In an embodiment the strain is a spore-forming strain. The strain may be purified or non-purified. The strain may be applied as a biologically pure culture or inoculum. The term "natamycin producing bacterial strain" as used herein also includes spores or spore-like structures of natamycin producing bacteria. The spores or spore-like structures themselves may be capable of producing natamycin. Alternatively, the spores or spore-like structures may not be capable of producing natamycin, but can develop into natamycin producing bacterial strains once conditions are favorable.

In an embodiment the natamycin producing bacterial strain is selected from the group consisting of a *Streptomyces natalensis* strain, a *Streptomyces gilvosporeus* strain, a *Streptomyces chattanoogensis* strain, and a *Streptomyces lydicus* strain. Methods for producing natamycin producing bacterial strains are known in the art, for example in WO 93/03171, WO 93/03169, WO 2004/087934, Martin and McDaniel (1977), Chen *et al.* (2008), Farid *et al.* (2000), EI-Enshasy *et al.* (2000b), He *et al.* (2002), and Liang *et al.* (2008). *Streptomyces natalensis* strains include, but are not limited to, the following strains: ATCC27448, BCRC 15150, CBS 668.72, CBS 700.57, CCRC 15150, CCTM La 2923, CECT 3322, CGMCC 100017, CGMCC 100019, DSM 40357, F ATCC27448, Hoogerheide strain KNGSF, IFO 13367, ISP 5357, JCM 4693, JCM 4795, KCC 693, KCC S-0693, KCC S-0795, KCCS-0693, KCCS-0795, KNGS strain F, NBRC 13367, NCIB 10038, NCIM 2933, NCIM 5058, NCIMB 10038, NRRL 2651, NRRL B-2651, NRRL B-5314, RIA 1328, RIA 976, VKM Ac-1175, VKM Ac-1175. Furthermore, the *Streptomyces natalensis* strains as described in the examples can be used in the present invention. In a preferred embodiment, *Streptomyces natalensis* strains are used in the present invention. In an even more preferred embodiment, the *Streptomyces natalensis* strains with the internal coding DS73870 and DS73871 are used in the present invention. The strains were deposited under the terms of the Budapest Treaty with the Centraal Bureau voor Schimmelcultures (CBS), Utrecht, Netherlands, on May 20, 2014. S. *natalensis* strain DS73870 has been deposited as strain CBS 137965. S. *natalensis* strain DS73871 has been deposited as strain CBS 137966.

*Streptomyces gilvosporeus* strains include, but are not limited to, the following strains: A-5283, ATCC13326, NRRL B-5623. *Streptomyces chattanoogensis* strains include, but are not limited to, the following strains: AS 4.1415, ATCC 13358, ATCC 19739, BCRC 13655, Burns J-23, CBS 447.68, CBS 477.68, CCRC 13655, CCTM La 2922, CECT 3321, CGMCC 100020, CGMCC 4.1415, CUB 136, DSM 40002, DSMZ 40002, Holtman J-23, IFO 12754, ISP 50002, ISP 5002, J-23, JCM 4299, JCM 4571, KCC S-0299, KCC S-0571, KCCS-0571, KCCS-0299, KCTC 1087, Lanoot R-8703, LMG 19339, NBRC 12754, NCIB 9809, NCIMB 9809, NRRL B-2255, NRRL-ISP 5002, R-8703, RIA 1019, VKM Ac-1775. *Streptomyces lydicus* strains include, but are not limited to, the following strains: CGMCC No.1653.

To control fungal plant pathogens and thus to enhance plant growth and crop yield, the natamycin producing bacterial strain is applied to the plant in an amount effective for controlling fungal diseases. The strain is effective when delivered at a concentration of 10³-10¹¹ colony forming units (cfu) per gram. For application on a plant from 0.1 to 10,000 g/ha can be used. The natamycin producing bacterial strain can be applied to the plant by for instance dipping, spraying, irrigating, watering, dusting, drenching, foaming, painting, sprinkling, or spreading-on. The bacterial strain can be applied in liquid form for instance as a suspension or emulsion. Alternatively, when in dry form for instance as a granule, pellet or powder, the natamycin producing bacterial strain can first be mixed with a suitable liquid carrier and then applied to the plant.

The natamycin producing bacterial strain can be used to control fungal plant pathogens on different types of plants including, but not limited to, corn, maize, triticale, peanut, flax, canola, rape, poppy, olive, coconut, grasses, soy, cotton, beet, (e.g. sugar beet and fodder beet), rice (any rice may be used, but is preferably selected from the group consisting of *Oryza sativa* sp. japonica, *Oryza sativa* sp. javanica, *Oryza sativa* sp. indica, and hybrids thereof), sorghum, millet, wheat, durum wheat, barley, oats, rye, sunflower, sugar cane, turf, pasture, alfalfa, or tobacco. It can also be used to control fungal plant pathogens on fruit plants including, but not limited to, rosaceous fruit, for example apples and pears; stone-fruits, for example peaches, nectarines, cherries, plums and apricots; citrus fruit, for example, oranges, grapefruit, limes, lemons, kumquats, mandarins and satsumas; nuts, for example pistachios, almonds, walnuts, coffee, cacao and pecan nuts; tropical fruits, for example, mango, papaya, pineapple, dates and bananas; and grapes; and vegetables including, but not limited to, leaf vegetables, for example endives, lambs lettuce, rucola, fennel, globe (head lettuce) and loose-leaf salad, chard, spinach and chicory; brassicas, for example, cauliflower, broccoli, Chinese cabbage, kale (winter kale or curly kale), kohlrabi, brussel sprouts, red cabbage, white cabbage and savoy; fruiting vegetables, for example, aubergines, cucumbers, paprika, marrow, tomatoes, courgettes, melons, watermelons, pumpkins and sweet corn; root vegetables, for example celeriac, turnip, carrots, swedes, radishes, horse radish, beetroot, salsify, celery; pulses, for example, peas and beans; and bulb vegetables, for example leeks, garlic and onions. The natamycin producing bacterial strain can also be used for the treatment of ornamental plants, for example, pansy, impatiens, petunia, begonia, Lisianthus, sunflower, ageratum, chrysanthemum and geranium.

The natamycin producing bacterial strain can be applied to a plant as such, *i.e.* without diluting or additional components present. However, the natamycin producing bacterial strain is typically applied in the form of a composition. So, in an embodiment the present invention relates to a composition comprising a natamycin producing bacterial strain and an agriculturally acceptable carrier. The composition may be a ready-to-use composition or a concentrate which has to be diluted before use. Preferably, the composition comprises an agriculturally acceptable carrier. The term "agriculturally acceptable carrier" as used herein means an inert, solid or liquid, natural or synthetic, organic or inorganic substance which is mixed or combined with the active agent, e.g. the natamycin producing bacterial strain, for better applicability on plants and parts thereof. It covers all carriers that are ordinarily used in (bio)fungicide formulation technology including, but not limited to, water, protective colloids, binders, salts, buffers, diluents, minerals, fillers, colorants, defoamers, adhesives, fixatives, tackifiers, resins, preservatives, stabilizers, fertilizers, anti-oxidation agents, gene activators, thickeners, plasticizers, siccatives, surfactants, dispersants, alcohols, complex formers, wetting agents, waxes, solvents, emulsifiers, mineral or vegetable oils, sequestering agents, and derivatives and/or mixtures thereof. The compositions may be mixed with one or more solid or liquid carriers and prepared by various means, e.g. by homogeneously mixing or blending the strain with suitable carriers using conventional formulation techniques. Depending on the type of composition that is prepared, further processing steps such as for instance granulation may be required.

In an embodiment the bacterial strain may be present at a level of 10³-10¹⁰ cfu/g carrier. In an embodiment the natamycin producing bacterial strain is present in 1% (w/w) to 99% (w/w) by weight of the entire composition, preferably 10% (w/w) to 75% (w/w). The present invention therefore also relates to a composition comprising a natamycin producing bacterial strain and an agriculturally acceptable carrier.

The natamycin producing bacterial strain can be applied simultaneously or in succession with other compounds to a plant. Examples of such compounds are fertilizers, growth regulators, (micro)nutrients, herbicides, rodenticides, miticides, bird repellents, attractants, insecticides, fungicides, acaricides, sterilants, bactericides, nematicides, mollusicides, or mixtures thereof. If desired, these other compounds may also comprise agriculturally acceptable carriers. If applied simultaneously, the natamycin producing bacterial strain and the other compound can be applied as one composition or can be applied as two or more separate compositions. If applied in succession, the natamycin producing bacterial strain can be applied first followed by the other compound or the other compound can be applied first followed by the natamycin producing bacterial strain. When the natamycin producing bacterial strain and the other compound are applied sequentially, the time between both applications may vary from e.g. 10 minutes to 100 days.

The present invention is also concerned with the use of a natamycin producing bacterial strain as a plant growth enhancer and/or crop yield enhancer. Of course, a composition according to the present invention can also be used as a plant growth enhancer and/or crop yield enhancer.

In an embodiment the composition of the present invention further comprises at least one further antimicrobial compound. The antimicrobial compound may be an antifungal compound or a compound to combat insects, nematodes, mites and/or bacteria. Of course, the compositions according to the invention may also comprise two or more of any of the above antimicrobial compounds. Compound as used herein also includes another antimicrobial strain. Preferably, the strain is an antimicrobial bacterial strain.

Compositions according to the invention may have a pH of from 1 to 10, preferably of from 2 to 9, more preferably of from 3 to 8 and most preferably of from 4 to 7. They may be solid, e.g. powder compositions, or may be liquid. The compositions of the present invention can be aqueous or non-aqueous ready-to-use compositions, but may also be aqueous or non-aqueous concentrated compositions/suspensions or stock compositions, suspensions and/or solutions which before use have to be diluted with a suitable diluent such as water or a buffer system. The compositions of the present invention can also have the form of concentrated dry products such as e.g. powders, granulates and tablets. They can be used to prepare compositions for immersion or spraying of plants and/or crops.

The natamycin producing bacterial strain and any other compound can be present in a kit of parts. The two or more components of the kit can be packaged separately. As such, kits include one or more separate containers such as vials, cans, bottles, pouches, bags or canisters, each container containing a separate component for an agrochemical composition. The components of the kit may be either in dry form or liquid form in the package. If necessary, the kit may comprise instructions for dissolving the compounds. In addition, the kit may contain instructions for applying the compounds.

### EXAMPLES

### Example 1

### Selection of natamycin producing Streptomyces natalensis strains

The following eight natamycin producing *Streptomyces natalensis* strains were selected from an internal culture collection and tested for their antifungal activity in an *in vitro* experiment: DS10599, DS73309, DS10601, DS73871, DS73870, DS73311, DS73352 and DS73312.

Three fungal plant pathogens were tested against the selected S. *natalensis* strains: *Fusarium oxysporum* f.sp. *lycopersici* (CBS414.90), *Colletotrichum gloeosporioides* (CBS272.51) and *Alternaria alternata* (CBS103.33). *Fusarium oxysporum* f.sp. *lycopersici* is a soil borne fungus causing yield loss in *e.g.* tomato crops (Fusarium Wilt). *Colletotrichum gloeosporioides* causes *e.g.* anthracnose, a plant disease recognizable by dark brown lesions on leaves and fruits. *Alternaria alternata* is a worldwide occurring saprophyte that can cause phytopathogenic reactions in economically important host plants. The selected S. *natalensis* strains were tested against these fungal plant pathogens as described below.

Frozen vials (glycerol stocks) or freeze dried tubes from the selected S. *natalensis* strains were transferred to 100 ml baffled Erlenmeyer flasks containing 20 ml of Yeast Malt Extract broth (YME). Freeze dried tubes were resuspended in physiological saline and stored 1 hour at room temperature before they were transferred to the medium. The Erlenmeyer flasks were incubated in an incubator shaker for 3 days at 28°C and 180 rpm.

For each strain, 200 µl of liquid media was transferred to YME agar plates (90 mm petridishes containing 20 ml medium) and dispersed with the use of a sterile spreader onto the surface of the medium. Subsequently, the agar plates were incubated for 4 days at 28°C to enhance full colony coverage of the plate surface.

The selected fungi were plated directly from a glycerol stock (200 µl) to YME agar and subsequently incubated for 4 days at 28°C.

The bio-assay was done as follows. Overgrown *Streptomyces* and fungi plates made as described above were used to produce agar plugs. The plugs were made by cutting out the agar by using a sterile cork-borer (11 mm diameter) and subsequently removing it by a pre-sterilized spatula. Freshly produced non-inoculated YME agar plates were marked with a line on the back of the petridish. This line with a fixed length of 4 cm was placed in the middle of the petridish. Subsequently, a *Streptomyces* inoculated agar plug was transferred to the fresh YME agar plate at the left end of the line. The above-described "plug transfer" step was repeated for the respective fungi. The respective fungi plugs were placed at the right end of the line on the same YME agar plate. Each *Streptomyces* strain was challenged in triplicate against each fungus. For each tested fungus, a control sample was taken (in triplicate) by using a non-inoculated agar plug that was placed on the left side of the petridish. Next, the plates were placed with the plugs on top in an incubator. Subsequently, the plates were stored at 28°C for 7 days. After 7 days the radius of the fungal colony was measured in the direction of the opposite agar plug (*Streptomyces* sample). This step was repeated for the control sample. The inhibition zone (in percentage) was calculated by using the following formula: 100% * (r₀-r₁)/r₀, wherein r₀ is the radius (in mm, corrected for the plug radius) of the fungal colony from the control sample and wherein r₁ is the radius (in mm, corrected for the plug radius) of the fungal colony from the *Streptomyces* inhibited sample.

The results demonstrate that the fungal growth towards the natamycin producing *S. natalensis* strains was significantly inhibited. All strains outperformed the control sample without *S*. *natalensis* and the average inhibition varied from 53% to 66% depending on the tested strains (see Table 1). The results clearly demonstrate that *S*. *natalensis* strains have the potential to inhibit different species of fungal plant pathogens.

*S*. *natalensis* with the internal coding DS73870 and DS73871 were selected for further research. The strains were deposited under the terms of the Budapest Treaty with the Centraal Bureau voor Schimmelcultures (CBS), Utrecht, Netherlands, on May 20, 2014. *S. natalensis* strain DS73870 has been deposited as strain CBS 137965. *S. natalensis* strain DS73871 has been deposited as strain CBS 137966.

### Example 2 (not according to the invention)

### Antifungal activity of natamycin producing Streptomyces strains

The following natamycin producing *Streptomyces* strains were tested for their antifungal activity in an *in vitro* experiment: *Streptomyces natalensis* ATCC-27448 (type strain), *Streptomyces natalensis* DS73871, *Streptomyces natalensis* DS73870 and *Streptomyces chattanoogensis* ATCC-19673.

Five fungal plant pathogens were tested against the selected Streptomyces strains: *Fusarium oxysporum* f.sp. *lycopersici* (CBS414.90), *Colletotrichum gloeosporioides* (CBS272.51), *Alternaria alternata* (CBS103.33), *Aspergillus niger* (ATCC16404) and *Botrytis cinerea* (CBS156.71). *Aspergillus niger,* also known as the black mould, is one of the most common *Aspergillus* species. This ubiquitous soil inhabitant is responsible for serious losses in different types of crops, such as: onions (black rot), grapes (fruit rot) and peanuts (crown rot). *Botrytis cinerea* is the causing agent of grey mould disease. This disease is recorded in a wide range of crops and has a high economic impact. For plant diseases related to *Fusarium oxysporum* f.sp. *lycopersici, Colletotrichum gloeosporioides* and *Alternaria alternata,* see Example 1. The selected *S. natalensis* strains were tested against these fungal plant pathogens as described below.

Frozen vials (glycerol stocks) from the selected *S. natalensis* strains were transferred to 100 ml baffled Erlenmeyer flasks containing 20 ml of Yeast Malt Extract broth (YME). The Erlenmeyer flasks were incubated in an incubator shaker for 3 days at 28 °C and 180 rpm.

For each strain, 200 µl of liquid media was transferred to YME agar plates and incubated for 4 days at 28°C to enhance full colony coverage of the plate surface.

*Botrytis cinerea* was plated directly from glycerol stock (100 µl) to YME agar and subsequently incubated for 9 days at 28°C. All other selected fungi were plated directly from a glycerol stock (200 µl) to YME agar and subsequently incubated for 4 days at 28°C.

The bio-assay was done according to the procedure described in Example 1, with the alteration that all variables were tested in five-fold. The plates were stored at 28°C for 7 days or 11 days, depending on the fungal colony growth of the control samples. After incubation, the radius of the fungal colony was measured according to the method described in Example 1.

The results demonstrate that the fungal growth towards the natamycin producing strains (*Streptomyces natalensis* & *Streptomyces chattanoogensis*) was inhibited in all tested samples compared to the control samples. The average inhibition varied from 2% to 78% depending on the tested strains (see Table 2). Therefore, these results clearly demonstrate that natamycin producing *Streptomyces* strains have the potential to inhibit different species of fungal plant pathogens.

The *S. natalensis* strains showed a higher reduction in the development of the fungal radius compared to the *S. chattanoogensis.* Furthermore, the *S. natalensis* strains DS73870 and DS73871 clearly outperformed the *S. natalensis* ATCC-27448 strain (= type strain). Depending on the tested fungal strains, the average inhibition of *S. natalensis* ATCC-27448 varied between 26% and 53%, whereas the average inhibition of *S. natalensis* DS73870 and DS73871 varied between 59% and 78% (see Table 2).

### Example 3 (not according to the invention)

### Antifungal activity of natamycin producing Streptomyces natalensis strains DS73871 & DS73870 against Verticillium albo-atrum.

In another experiment, the natamycin producing *Streptomyces natalensis* DS73871 and *Streptomyces natalensis* DS73870 were tested for their antifungal activity against *Verticillium albo-atrum* (CBS321.91).

*Verticillium albo-atrum* is associated with Verticillium wilt. This soil borne fungus can cause serious harvest losses on a wide variety of crops, mainly in the cooler climate regions.

The experiment was done according to the method described in Example 1 with the proviso that all variables were tested in five-fold. For the production of fungal plugs, *Verticillium albo-atrum* (CBS321.91) was plated directly from a glycerol stock (200 µl) to YME agar and subsequently incubated for 4 days at 28°C.

The fungal radius of *Verticillium albo-atrum* towards the natamycin producing *Streptomyces natalensis* strains DS73871 and DS73870 was reduced by respectively 44% and 45% at day 11. After 25 days the inhibition zone was even further reduced to 76% and 71% for strains DS73871 and DS73870, respectively (see Table 3).

These results clearly demonstrate that *S. natalensis* DS73870 and DS73871 have the ability to inhibit *Verticillium albo-atrum.*

### Example 4 (not according to the invention)

### Antifungal activity of natamycin producing Streptomyces natalensis DS73871 & DS73870 against Cercospora zeae-maydis.

In another experiment, the natamycin producing *Streptomyces natalensis* strains DS73871 and DS73870 were tested for their antifungal activity against *Cercospora zeae-maydis* (CBS117757). *Cercospora zeae-maydis* causes "Grey leaf spot", one of the most important foliar diseases on maize.

The experiment was done according to the method described in Example 1, with the only exception that the petridishes for "bioassay" (containing both bacterial and mould plugs) were stored for 28 days at 28°C instead of 7 days.

The results (see Table 4) clearly demonstrate the inhibitory effect of *S. natalensis* strains DS73870 and DS73871 on the growth of *Cercospora zeae-maydis.* The radius of the fungal colony was decreased by 84% and 63% towards the bacterial strain for *S. natalensis* DS73871 and *S. natalensis* DS73870, respectively.

### Example 5 (not according to the invention)

### Antifungal activity of natamycin producing Streptomyces natalensis DS73871 and DS73870 compared to other Streptomyces sp. against Colletotrichum gloeosporioides

This example describes the comparison of antifungal activity of natamycin producing strains *Streptomyces natalensis* DS73871 & DS73870 and several non-natamycin producing *Streptomyces* sp. (*S. griseus, S. griseoviridis* and *S*. *rochei*) against the fungal plant pathogen *Colletotrichum gloeosporioides.* The analysed non-natamycin producing strains were requested from the following public depositories: S. *griseus* (NRRLB1354), *S*. *griseoviridis* (NRRL2427) and *S*. *rochei* (CBS939.68).

The experiment was done according to the method described in Example 1 with the proviso that all variables were tested in five-fold and the pre-incubation time (culturing broth) was enhanced from 3 days to 4 to allow full growth of all strains. The fungal inhibition on the colony radius of *Colletotrichum gloeosporioides* (CBS272.51) was determined after 6 days of incubation at 28°C.

The results can be found in Table 5. The fungal radius of *C. gloeosporioides* was clearly reduced towards the opposing *Streptomyces* species in all samples compared to the control (no *Streptomyces* sp.). Furthermore, both *Streptomyces natalensis* DS73871 and DS73870 showed a stronger inhibitory effect (respectively 56% and 54%) compared to the non-natamycin producing Streptomyces species (between 7% and 33%).

### Example 6 (not according to the invention)

### Antifungal activity of natamycin producing Streptomyces natalensis DS73871 & DS73870 compared to other Streptomyces sp. against Fusarium oxysporum f.sp. lycopersici.

In another experiment, the natamycin producing strains *Streptomyces natalensis* DS73871 & DS73870 were compared to the non-natamycin producing *Streptomyces* species: *S noursei* and *S*. *griseus* for their antifungal activity against *Fusarium oxysporum* f.sp. *lycopersici.*

All three selected *Streptomyces* species are well described in literature for their ability to produce antifungal components. The analysed non-natamycin producing strains were requested from the following public depositories: *Streptomyces noursei* (CBS240.57) and *S. griseus* (NRRLB1354).

The experiment was done according to the method described in Example 1 with the proviso that all variables were tested in five-fold and the pre-incubation time (culturing broth) was enhanced from 3 days to 4 to allow full growth of all strains. The fungal inhibition of the *Fusarium oxysporum* f.sp. *lycopersici* (CBS414.90) colony radius was determined after 7 days of incubation at 28°C.

The colony growth of *Fusarium oxysporum* f.sp. *lycopersici* (CBS414.90) can be found in Table 6. The average inhibition zone was clearly reduced when challenged against *Streptomyces* species (between 11% and 60%). However the fungal inhibition of the natamycin producing *Streptomyces* species (60% and 54% for DS73870 and DS73871, respectively) was clearly stronger compared to the non-natamycin producing *Streptomyces* species (11% and 32% for *S. griseus* and *S*. *noursei,* respectively).

### Example 7 (not according to the invention)

### Antifungal activity Natamycin producing Streptomyces natalensis ATCC 27448 compared to pure natamycin against Colletotrichum gloeosporioides.

In another experiment, the natamycin producing strain *Streptomyces natalensis* ATCC 27448 was cultured on YME agar plates to determine the natamycin concentration in the agar media. In a next step, the bioactivity of the *Streptomyces natalensis* strain was compared to a concentration range of pure natamycin (dissolved in methanol) against *Colletotrichum gloeosporioides.* This experiment was conducted following the protocol described below.

A frozen vial containing *Streptomyces natalensis* ATCC 27448 culture media was transferred to a 100 ml baffled Erlenmeyer flask containing 20 ml of Yeast Malt Extract broth (YME). The Erlenmeyer flask was incubated in an incubator shaker for 4 days at 28°C and 180 rpm. 200 µl of the full grown culture broth was transferred (in duplo) to 90 mm petridishes containing exactly 20 ml of YME agar (YMEA). Subsequently, the inoculum was dispersed with the use of a sterile spreader onto the surface of the media. The plates were incubated for 4 days at 28°C to enhance full colony coverage. After incubation the plates were freeze-dried (Alpha 2-4 LD Freeze dryer, Christ). The freeze-dried content of each agar plate was transferred to a volumetric flask and filled with preheated MilliQ (50°C) to a final volume of 500 ml.

This solution was stirred for approximately 30 minutes, centrifuged (8 minutes, 21,000 rcf) and the supernatant was subsequently tested for its natamycin concentration. The natamycin concentration was determined by using a well-known literature based method (HPLC-UV) and calculated back to the average concentration in the media plate.

In a next step, the full grown culture of *Streptomyces natalensis* ATCC-27448 was reproduced on YMEA (20 ml media in each 90 mm petridish), using the protocol described above. This culture was used for a bio-assay against *Colletotrichum gloeosporioides* (CBS272.51) using the bio-assay as described in Example 1. Next to the *Streptomyces natalensis* ATCC-27448 inoculated samples, control samples were taken by using a non-inoculated agar plug.

In parallel, YMEA plates were produced containing different concentrations of pure natamycin. Therefore, natamycin stock solutions were prepared by dissolving natamycin (Analytical grade, DSM Food Specialties, Delft, The Netherlands) into methanol (Merck, gradient grade for liquid chromatography, ≥99,9 %). Subsequently, the natamycin stock solutions were added to liquid YME agar (45°C, corrected for the addition of methanol by lowering the water content) in a 1:19 ratio and mixed thoroughly through the media. The final natamycin concentrations in the agar were respectively: 500, 375, 250, 175, 100, 75, 50, 25, 10 and 0 ppm natamycin. The 0 ppm natamycin YMEA plates contained 5% (w/w) methanol only. The liquid YMEA was transferred to petridishes (20 ml media in each 90 mm petridish, done in threefold). After solidification, the YMEA plates were used for the bio-assay method against *Colletotrichum gloeosporioides* (CBS272.51) as described in Example 1. All samples were processed within the same day.

After 7 days of incubation at 28°C the fungal radius and inhibition zone for *Colletotrichum gloeosporioides* (CBS272.51) were determined (see method described in Example 1).

The average concentration of natamycin that is produced by *Streptomyces natalensis* ATCC-27448 during pre-incubation in the agar media, was measured to be less than 10 ppm (however, not 0 ppm). By dissolving 10 ppm pure natamycin directly into an agar plug, the inhibition zone of *Colletotrichum gloeosporioides* was not as high compared to the inhibition zone produced by the *Streptomyces natalensis* ATCC-27448 agar plug (see Table 7). Similar inhibition zones were matched at a much higher concentration rate (approximately 375 ppm).

### Example 8

### Treatment of corn plants

A glycerol stock of spores of natamycin producing bacterial strain (ATCC27448) was provided. An aliquot from the stock was used to culture the strain in a 11 flask with 200 mL YEME liquid media without sucrose (see www.elabprotocols.com) and glass beads (to break up aggregates). The culture flask was placed in a 200 rpm shaker at 28°C for 72 hours. The resulting suspension was transferred to a 250 ml tube and centrifuged at 8,000 rpm for 15 minutes. The resulting pellet was resuspended in sterile water to a final concentration of 200 mg/ml. This suspension was used as the strain inoculum for the below greenhouse experiment.

*Cercospora zeae-maydis* was cultured in 30% V8 juice agar plates and incubated for 2 weeks under diurnal light and 25°C (see Beckman and Payne, 1983). To prepare the inoculum for the greenhouse study, conidia were harvested by flooding the plates with 3 ml 0.01% Tween-20 and dislodging the spores gently with sterile pipet tips. This process was done twice. The resulting conidial suspension obtained about 10⁴ conidia/ml.

Corn seeds (Pioneer Hybrid 35F40) were surface sterilized by first rinsing them with 70% ethanol and then soaking them in a sodium hypochlorite solution. The resulting corn seeds were soaked in 0.5% NaClO + a drop of Tween-20 for 20 minutes. The seeds were then rinsed 5 times with sterile distilled water.

The seeds were sown in 3.25" x 3.25" x 3.25" pots containing coarse/expanded vermiculite (Therm-O-Rock West, Inc., AZ). Two seeds were sown in each pot. When the seedlings emerged, they were thinned out to leave one plant per pot.

The plants were allowed to grow in the greenhouse with the following conditions: 25°C day time temperature and 18°C night time temperature; 14 hours daylight and 10 hours dark. Full fertilizer was supplied to the plants during the entire growth period.

Forty-two (42) days after planting, treatments were initiated. The treatments applied were:
a) Treatment 1: Control;
b) Treatment 2: Plant inoculated with natamycin producing bacterial strain first, and 4 days later, inoculated with *Cercospora zeae-maydis;*
c) Treatment 3: Plant inoculated with *Cercospora zeae-maydis* first, and 4 days later, inoculated with natamycin producing bacterial strain;
d) Treatment 4: Plant inoculated with natamycin producing bacterial strain;
e) Treatment 5: Plant inoculated with *Cercospora zeae-maydis.*
Ten biological replicates were done per treatment.

The corn plants were inoculated with pathogen by spraying *Cercospora zeae-maydis* spore suspension directly on the leaves. The corn plants were then enclosed in a clear plastic canopy to maintain a relative high humidity around the plants. A high relative humidity is a requirement for the establishment of *Cercospora zeae-maydis* infection on corn leaves. The plastic covers were removed 5 days after treatment application.

The natamycin producing bacterial strain was applied by adding 2 ml of the strain in the root region of the plants. This is equivalent to 400 mg inoculum per pot.
The plants were monitored for five weeks after initiation of each treatment for disease development as well as growth promotion.

To evaluate if there were statistically significant differences in growth, the plant height (standing height method) was measured at 84 days after planting (DAP). The results show that the plants treated with the natamycin producing bacterial strain alone produced taller plants than any other treatment at 84 DAP (see Figure 1). This demonstrates that natamycin producing bacterial strains enhance plant growth.

### Example 9 (not according to the invention)

### Effect of plant treatment with Streptomyces natalensis DS73870 and DS73871 on lettuce grown in soil artificially infested with Rhizoctonia solani.

*Rhizoctonia solani* (CBS 323.84), a soil borne plant pathogen, was obtained from a 9 day old MEA media agar plate (incubation temperature 24°C) and dissolved in water. The fungal inoculum (50 ml/I soil) was mixed thoroughly through soil (90% peat, 10% sand). Seeding trays were filled with the inoculated soil (7.5 liter soil per seeding tray) and incubated for 2 days. Subsequently, the seeds were placed into the soil at a depth of approximately 1 cm.

The plant treatment started 4 days after seeding (seedling stage) and was repeated weekly for maximum of 3 weeks. For each plant treatment, a fresh *S. natalensis* culture broth was prepared. Therefore, a frozen vial from *Streptomyces natalensis* strains DS73870 was transferred to 100 ml baffled Erlenmeyer flasks containing 20 ml of Yeast Malt Extract broth (YME). The Erlenmeyer flasks were incubated in an incubator shaker for 3∼4 days at 28°C and 180 rpm (G24 Environmental Incubator Shaker, New Brunswick Scientific Co.). Subsequently, 2 ml of cultured broth was transferred to 500 ml baffled Erlenmeyer flasks containing 200 ml of YME broth. The media were incubated for another 4 days at 28°C and 180 rpm (Orbital Incubator Inr200-010V, Gallenkamp). The average bacterial count after incubation of the *S. natalensis* DS73870 samples was 7.1 (+/- 0,6) log CFU/ml.

The samples were shipped (under cooled conditions) to a greenhouse lab facility and processed within 24 hours. Therefore, the media containing *S. natalensis* DS73870 was 100-fold diluted in drinking water. From this diluted medium, 7 ml was added to each plant by watering the soil around the seedling or stem. Control samples were treated under the same conditions as for the *S. natalensis* treated samples, with the exception that only drinking water was added.

Each treatment was tested in 4 replicates. Each replicate consisted of 1 seeding tray containing 96 seeds. Trials were conducted according to EPPO guidelines PP 1/148(2), PP 1/135(3) and PP 1/152(4). The treatments were maintained under controlled greenhouse conditions and watered at set time intervals. The seedlings or plants were assessed weekly on: germination rate and disease severity for a maximum of 1 month after seeding.

The results of this study are summarized in Table 8 (germination rate) and Table 9 (plant disease severity). The observed number of unaffected plants was increased when plants were treated with *Streptomyces natalensis* DS73870 compared to treatment with sterile medium (control). Also, the amount of lettuce plants that were not germinated or died after germination (classified as not present) was exceedingly higher for the control samples compared to the *S. natalensis* treated samples. This trend was also observed for plant disease severity, in which *S. natalensis* DS73870 treated plants showed a clear reduction (see Table 9) when compared to the control.

In conclusion, by applying a natamycin producing *S. natalensis* strain to a plant, both plant growth and vitality of crops that are affected by fungal plant pathogens are enhanced.

**Table 1: Fungal radius of different plant pathogens tested against several Streptomyces natalensis strains on YME agar plates after 7 days of incubation at 28°C.**

| Tested fungus | Tested *S. natalensis* strain | Fungal radius (in mm) | Average inhibition zone (in %) |
|---|---|---|---|
| *Fusarium oxysporum* f.sp. *lycopersici* (CBS414.90) | Control (no *S. natalensis* strain) | 28.7 | 0 |
| | DS10599 | 13.3 | 53 |
| | DS10601 | 12.0 | 58 |
| | DS73871 | 11.3 | 60 |
| | DS73309 | 12.7 | 56 |
| | DS73311 | 13.3 | 53 |
| | DS73312 | 12.7 | 56 |
| | DS73870 | 11.3 | 60 |
| | DS73352 | 11.3 | 60 |
| *Colletotrichum gloeosporioides* (CBS272.51) | Control (no *S. natalensis* strain) | 39.3 | 0 |
| | DS10599 | 16.3 | 58 |
| | DS10601 | 15.7 | 60 |
| | DS73871 | 14.0 | 64 |
| | DS73309 | 16.0 | 59 |
| | DS73311 | 14.3 | 64 |
| | DS73312 | 15.0 | 62 |
| | DS73870 | 13.3 | 66 |
| | DS73352 | 13.7 | 65 |
| *Alternaria alternate* (CBS103.33) | Control (no *S. natalensis* strain) | 22.3 | 0 |
| | DS10599 | 9.3 | 58 |
| | DS10601 | 9.0 | 60 |
| | DS73871 | 8.3 | 63 |
| | DS73309 | 8.7 | 61 |
| | DS73311 | 8.0 | 64 |
| | DS73312 | 8.3 | 63 |
| | DS73870 | 8.7 | 61 |
| | DS73352 | 8.3 | 63 |

**Table 2: Fungal radius of different plant pathogens tested against several strains of natamycin producing Streptomyces species on YME agar plates after 7 or 11 days of incubation at 28°C.**

| Tested fungus | Days of incubation | Tested *Streptomyces* strain | Fungal radius (in mm) | Average inhibition zone (in %) |
|---|---|---|---|---|
| *Fusarium oxysporum* f.sp. *lycopersici* (CBS414.90) | 7 | Control (no *Streptomyces* strain) | 36.9 | 0 |
| | 7 | *S. chattanoogensis* ATCC 19673 | 33.9 | 8 |
| | 7 | *S. natalensis* ATCC27448 | 27.5 | 26 |
| | 7 | *S. natalensis* DS73871 | 14.8 | 60 |
| | 7 | *S. natalensis* DS73870 | 14.0 | 62 |
| *Colletotrichum gloeosporioides* (CBS272.51) | 7 | Control (no *Streptomyces* strain) | 42.2 | 0 |
| | 7 | *S. chattanoogensis* ATCC 19673 | 35.5 | 16 |
| | 7 | *S. natalensis* ATCC27448 | 30.2 | 28 |
| | 7 | *S. natalensis* DS73871 | 16.6 | 61 |
| | 7 | *S. natalensis* DS73870 | 17.4 | 59 |
| *Alternaria alternata* (CBS103.33) | 11 | Control (no *Streptomyces* strain) | 43.5 | 0 |
| | 11 | *S. chattanoogensis* ATCC 19673 | 38.3 | 12 |
| | 11 | *S. natalensis* ATCC27448 | 20.6 | 53 |
| | 11 | *S. natalensis* DS73871 | 9.9 | 77 |
| | 11 | *S. natalensis* DS73870 | 9.8 | 78 |
| *Aspergillus niger* (ATCC16404) | 7 | Control (no *Streptomyces* strain) | 43.4 | 0 |
| | 7 | *S. chattanoogensis* ATCC 19673 | 42.5 | 2 |
| | 7 | *S. natalensis* ATCC27448 | 28.2 | 35 |
| | 7 | *S. natalensis* DS73871 | 14.8 | 66 |
| | 7 | *S. natalensis* DS73870 | 15.7 | 64 |
| *Botrytis cinereal* (CBS156.71) | 11 | Control (no *Streptomyces* strain) | 7.7 | 0 |
| | 11 | *S. chattanoogensis* ATCC 19673 | 5.4 | 30 |
| | 11 | *S. natalensis* ATCC27448 | 5.1 | 33 |
| | 11 | *S. natalensis* DS73871 | 2.3 | 70 |
| | 11 | *S. natalensis* DS73870 | 2.0 | 74 |

**Table 3: Fungal radius of Verticillium albo-atrum (CBS321.91) tested against Streptomyces natalensis DS73870 and DS73871 on YME agar plates after 11 days and 25 days of incubation at 28°C.**

| Tested fungus | Days of incubation | Tested *S. natalensis* strain | Fungal radius (in mm) | Average inhibition zone (in %) |
|---|---|---|---|---|
| *Verticillium alboatrum* | 11 | Control (no *S. natalensis* strain) | 7.1 | 0 |
| | 11 | *S. natalensis* DS73871 | 4.0 | 44 |
| | 11 | *S. natalensis* DS73870 | 3.9 | 45 |
| | 25 | Control (no *S. natalensis* strain) | 17.5 | 0 |
| | 25 | *S. natalensis* DS73871 | 4.2 | 76 |
| | 25 | *S. natalensis* DS73870 | 5.0 | 71 |

**Table 4: Fungal radius of Cercospora zeae-maydis (CBS117757) tested against Streptomyces natalensis DS73870 and DS73871 on YME agar plates after 28 days of incubation at 28°C.**

| Tested fungus | Tested *S*. *natalensis* strain | Fungal radius (in mm)* | Average inhibition zone (in %) |
|---|---|---|---|
| *Cercospora zeae-maydis* | Control (no *S. natalensis* strain) | 6.3 | 0 |
| | *S. natalensis* DS73871 | 1.0 | 84 |
| | *S. natalensis* DS73870 | 2.3 | 63 |

**Table 5: Fungal radius of Colletotrichum gloeosporioides (CBS272.51) tested against several Streptomyces sp. on YME agar plates after 6 days of incubation at 28°C.**

| Tested fungus | Days of incubation | Tested *Streptomyces* strain | Fungal radius (in mm) | Average inhibition zone (in %) |
|---|---|---|---|---|
| *Colletotrichum gloeosporioides* | 6 | Control (no *Streptomyces* strain) | 37.9 | 0 |
| | 6 | *S. natalensis* DS73870 | 17.4 | 54 |
| | 6 | *S. natalensis* DS73871 | 16.7 | 56 |
| | 6 | *S. griseus* NRRLB1354 | 25.4 | 33 |
| | 6 | *S. griseoviridis* NRRL2427 | 35.1 | 7 |
| | 6 | *S. rochei* CBS939.68 | 34.2 | 10 |

**Table 6: Fungal radius of Fusarium oxysporum f.sp. lycopersici (CBS414.90) tested against several Streptomyces sp. on YME agar plates after 7 days of incubation at 28°C.**

| Tested fungus | Days of incubation | Tested *Streptomyces* strain | Fungal radius (in mm) | Average inhibition zone (in %) |
|---|---|---|---|---|
| *Fusarium oxysporum* f.sp. *lycopersici* | 7 | Control (no *Streptomyces* strain) | 37.0 | 0 |
| | 7 | *S. natalensis* DS73870 | 15.0 | 60 |
| | 7 | *S. natalensis* DS73871 | 17.0 | 54 |
| | 7 | *S. griseus* NRRLB1354 | 33.1 | 11 |
| | 7 | *S. noursei* CBS240.57 | 25.1 | 32 |

**Table 7: Agar plug bio-assay. Fungal radius of Colletotrichum gloeosporioides (CBS272.51) tested against S. natalensis ATCC27448 and different ratios of pure natamycin dissolved in methanol (5% w/w). Results produced on YME agar plates after 7 days of incubation at 28°C.**

| Tested fungus | Plug content | Fungal radius (in mm) | Average inhibition zone (in %) |
|---|---|---|---|
| *Colletotrichum gloeosporioides* | Control (sterile plug) | 46.3 | 0 |
| | *S. natalensis* ATCC27448 (containing <10 ppm natamycin derived from pre-incubation) | 29.3 | 37 |
| | 0 ppm Natamycin* | 45.2 | 2 |
| | 10 ppm Natamycin* | 44.3 | 4 |
| | 25 ppm Natamycin* | 43.2 | 7 |
| | 50 ppm Natamycin* | 39.3 | 15 |
| | 75 ppm Natamycin* | 37.8 | 18 |
| | 100 ppm Natamycin* | 36.7 | 21 |
| | 175 ppm Natamycin* | 34.8 | 25 |
| | 250 ppm Natamycin* | 33.2 | 28 |
| | 375 ppm Natamycin* | 30.3 | 35 |
| | 500 ppm Natamycin* | 27.3 | 41 |

| | | | |
|---|---|---|---|
| * dissolved in YMEA containing 5% w/w methanol, given concentration is the concentration of the agar plug | | | |

**Table 8: Effect of plant treatment with Streptomyces natalensis DS73870 on the germination rate of Lettuce grown in soil artificially infested with Rhizoctonia solani.**

| Seed treatment | Day of measuremen t* | Germination rate (n = 96) | | | |
|---|---|---|---|---|---|
| | | Unaffected growth | Abnormal growth | Inhibited growth | Not present** |
| control (untreated) | 7 | 11.3 | 20.8 | 1.3 | 62.8 |
| *S. natalensis* DS73870 | | 13.0 | 19.5 | 1.8 | 61.5 |
| | | | | | |
| control (untreated) | 12 | 4.3 | 1.8 | 8.8 | 81.3 |
| *S. natalensis* DS73870 | | 11.0 | 2.0 | 8.3 | 74.8 |
| | | | | | |
| control (untreated) | 19 | 6.0 | 8.5 | 7.0 | 74.5 |
| *S. natalensis* DS73870 | | 10.0 | 7.5 | 8.3 | 70.0 |
| | | | | | |
| control (untreated) | 26 | 5.5 | 0.0 | 10.3 | 80.3 |
| *S. natalensis* DS73870 | | 8.3 | 0.0 | 13.3 | 74.5 |
| | | | | | |
| control (untreated) | 32 | 5.3 | 1.3 | 11.8 | 77.8 |
| *S. natalensis* DS73870 | | 10.5 | 2.5 | 10.0 | 73.0 |

| | | | | | |
|---|---|---|---|---|---|
| *measured from day of seeding (day 0) **Not germinated or deceased after germination | | | | | |

**Table 9: Effect of plant treatment with Streptomyces natalensis DS73870 on the plant disease severity of Lettuce grown in soil artificially infested with Rhizoctonia solani.**

| Plant treatment | Day of measurement* | Plant disease severity (n = 96) | | | | |
|---|---|---|---|---|---|---|
| | | Healthy | Light | Moderate | Severe | Not present** |
| control | 7 | 12.5 | 20.8 | 0.0 | 0.0 | 62.8 |
| DS73870 | | 14.8 | 19.5 | 0.0 | 0.0 | 61.5 |
| | | | | | | |
| control | 12 | 1.5 | 0.0 | 0.8 | 12.5 | 81.3 |
| DS73870 | | 6.0 | 0.0 | 1.3 | 14.0 | 74.8 |
| | | | | | | |
| control | 19 | 13.3 | 1.0 | 1.8 | 5.5 | 74.5 |
| DS73870 | | 19.8 | 2.8 | 0.5 | 2.8 | 70.0 |
| | | | | | | |
| control | 26 | 11.0 | 0.0 | 0.0 | 4.8 | 80.3 |
| DS73870 | | 17.3 | 0.0 | 0.0 | 4.3 | 74.5 |
| | | | | | | |
| control | 32 | 14.5 | 2.3 | 0.3 | 1.3 | 77.8 |
| DS73870 | | 20.3 | 1.3 | 0.3 | 1.3 | 73.0 |

| | | | | | | |
|---|---|---|---|---|---|---|
| *measured from day of seeding (day 0) **Not germinated or deceased after germination | | | | | | |

### REFERENCES

Beckman PM and Payne GA (1983), Cultural techniques and conditions influencing growth and sporulation of Cercospora zeae-maydis and lesion development in corn. Phytopathology 73:286-289.
Chen GQ, Lu FP and Du LX (2008), Natamycin production by Streptomyces gilvosporeus based on statistical optimization. J. Agric. Food Chem. 56:5057-5061.
Farid MA, EI-Enshasy HA, EI-Diwany Al and EI-Sayed, EA (2000), Optimization of the cultivation medium for natamycin production by Streptomyces natalensis. J. Basic Microbiol. 40: 157-166.
EI-Enshasy HA, Farid MA and EI-Sayed, EA (2000), Influence of inoculum type and cultivation conditions on natamycin production by Streptomyces natalensis. J. Basic Microbiol. 40: 333-342.
He YL, Wu JG, Lu FP and Du LX (2002) Fed-batch fermentation to improve the yield of natamycin. Med. Biotechnol. 9:224-226.
Liang JG, Xu ZN, Liu TF, Lin HP and Cen PL (2008), Effects of cultivation conditions on the production of natamycin with Streptomyces gilvosporeus LK-196. Enzyme Microb. Technol. 42:145-150.
Martin JF and McDaniel LE (1977), Production of polyene macrolide antibiotics. Advances in Appl. Microbiol. 21:1-52.

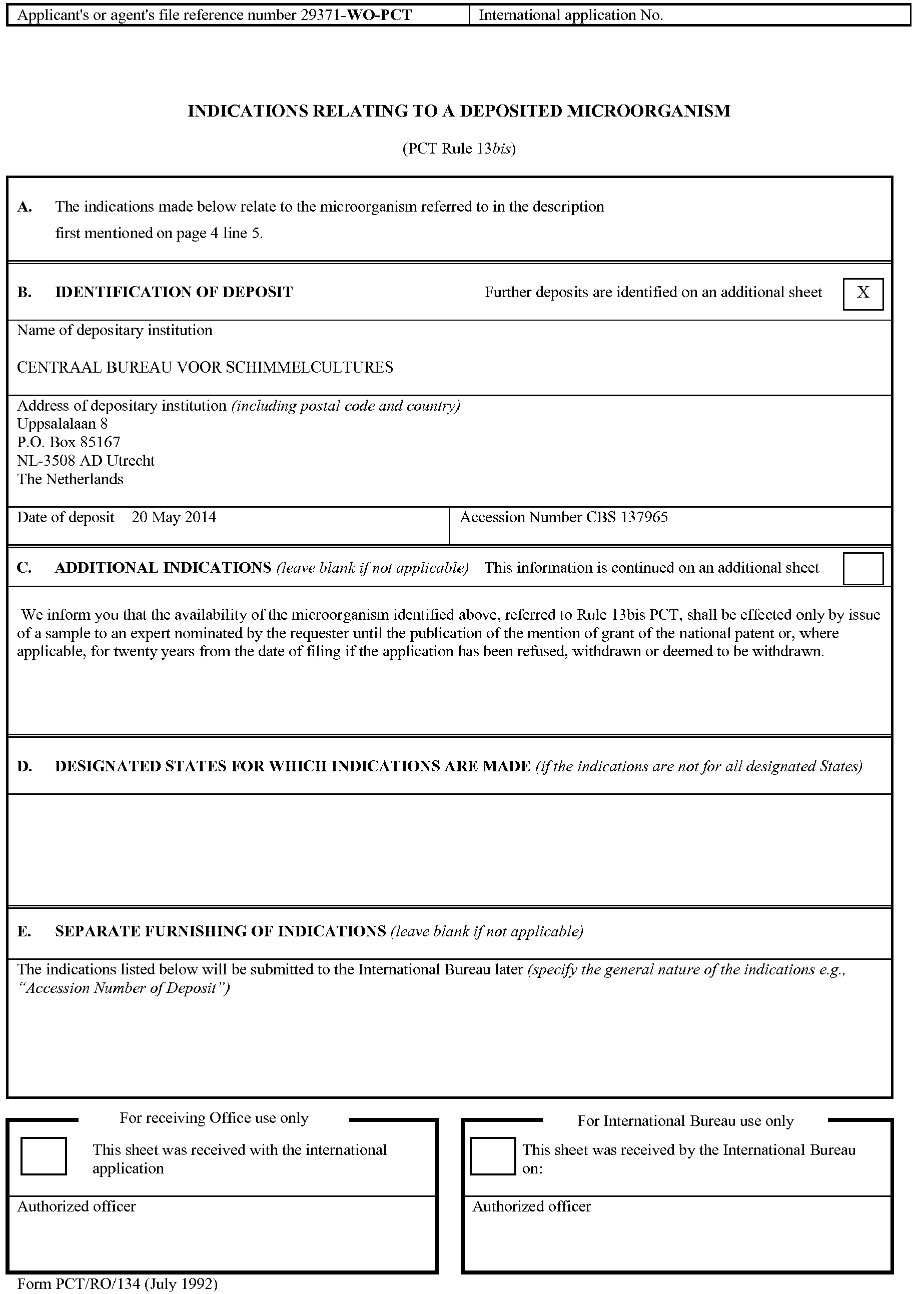

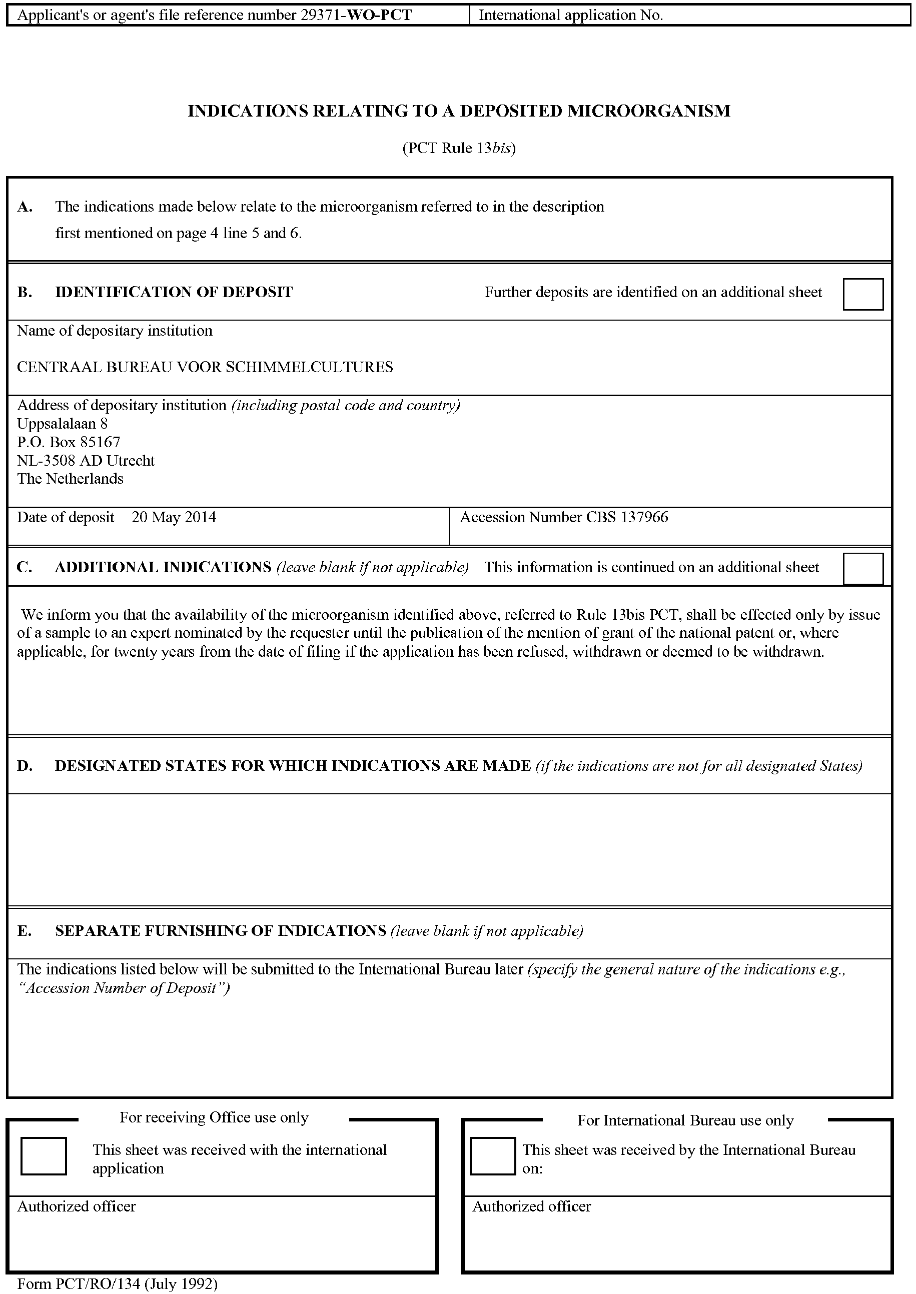

## Claims

1. A method for enhancing plant growth, crop yield or both, the method comprising the step of applying at least one natamycin producing bacterial strain to a plant.

2. The method according to claim 1, wherein the strain is applied in the form of a composition comprising an agriculturally acceptable carrier.

3. The method according to claim 2, wherein the composition comprises 10³-10¹⁰ cfu/g carrier.

4. The method according to any one of the claims 1 to 3, wherein the natamycin producing bacterial strain is selected from the group consisting of a *Streptomyces natalensis* strain, a *Streptomyces gilvosporeus* strain, and a *Streptomyces chattanoogensis* strain.

5. Use of at least one natamycin producing bacterial strain as a plant growth enhancer and/or crop yield enhancer.

## Patentansprüche

1. Verfahren zum Fördern des Pflanzenwachstums, des Ernteertrags oder von beidem, wobei das Verfahren den Schritt des Ausbringens von mindestens einem natamycinproduzierenden Bakterienstamm auf eine Pflanze umfasst.

2. Verfahren nach Anspruch 1, wobei der Stamm in Form einer Zusammensetzung umfassend einen landwirtschaftlich annehmbaren Träger ausgebracht wird.

3. Verfahren nach Anspruch 2, wobei die Zusammensetzung 10³-10¹⁰ KBE/g Träger umfasst.

4. Verfahren nach einem der Ansprüche 1 bis 3, wobei der natamycinproduzierende Bakterienstamm aus der Gruppe bestehend aus einem *Streptomyces-natalensis-Stamm,* einem *Streptomyces-gilvosporeus-Stamm* und einem *Streptomyceschattanoogensis-Stamm* ausgewählt ist.

5. Verwendung von mindestens einem natamycinproduzierenden Bakterienstamm als Pflanzenwachstumsförderer und/oder Ernteertragsförderer.

## Revendications

1. Méthode d'amélioration de la croissance de plantes, du rendement de cultures, ou des deux, la méthode comprenant l'étape consistant à appliquer au moins une souche bactérienne productrice de natamycine à une plante.

2. Méthode selon la revendication 1, dans laquelle la souche est appliquée sous la forme d'une composition comprenant un véhicule acceptable sur le plan agricole.

3. Méthode selon la revendication 2, dans laquelle la composition comprend 10³-10¹⁰ ufc/g de véhicule.

4. Méthode selon l'une quelconque des revendications 1 à 3, dans laquelle la souche bactérienne productrice de natamycine est choisie dans le groupe constitué par une souche de *Streptomyces natalensis,* une souche de *Streptomyces gilvosporeus,* et une souche de *Streptomyces chattanoogensis.*

5. Utilisation d'au moins une souche bactérienne productrice de natamycine comme améliorateur de la croissance de plantes et/ou améliorateur du rendement de cultures.
